# EUROPEAN PATENT APPLICATION

(11) **EP 1 810 657 A1**
(43) Date of publication of application: **25.07.2007**
(21) Application number: 05795892.8
(22) Date of filing: 21.10.2005
(51) Int. Cl.: A61K 8/00, A61Q 5/00, A61Q 5/02, A61Q 5/12

(54) **HAIR CARE PREPARATIONS**

(30) Priority: 22.10.2004 JP 2004308733
(71) Applicant: SHISEIDO COMPANY, LTD., Tokyo 104-8010 (JP)
(72) Inventor: GUENTERT, Miki, c/o SHISEIDO RESEARCH CENTER, Yokohama-shi, Kanagawa 2248558 (JP); HASHIMOTO, Katsuo, c/o SHISEIDO RESEARCH CENTER, Yokohama-shi, Kanagawa 2248558 (JP); AKUTSU, Takahiro, c/o SHISEIDO RESEARCH CENTER, Yokohama-shi, Kanagawa 2248558 (JP); KAWASOE, Tomoyuki, c/o SHISEIDO RESEARCH CENTER, Yokohama-shi, Kanagawa 2248558 (JP)
(74) Representative: Henkel, Feiler & Hänzel
(86) International application number: PCT/JP2005/019430
(87) International publication number: WO 2006/043676

(57) **Abstract**

The present invention intends to provide a hair cosmetic composition having an excellent hair protection effect and its long durability, and giving a moist feeling, softness and smoothness to the hair.

The aspect of the invention is a hair cosmetic composition comprising one or more amino-modified or ammonium-modified silicones represented by the following formula (I): 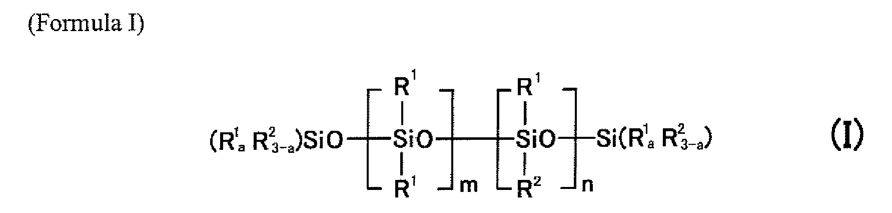 wherein each R¹ is independently a methyl group, a phenyl group or a hydroxyl group, each R² is independently a group shown by a formula R⁴Z, a is an integer of 0 to 3, each of m and n is a positive integer, a mean value of m+n is a range of from 4000 to 6000, and a mean value of n/m is a range of from 0.002 to 0.03,
wherein R⁴ is an alkylene group having a carbon number of 3 to 6, and Z is represented by the following formula: 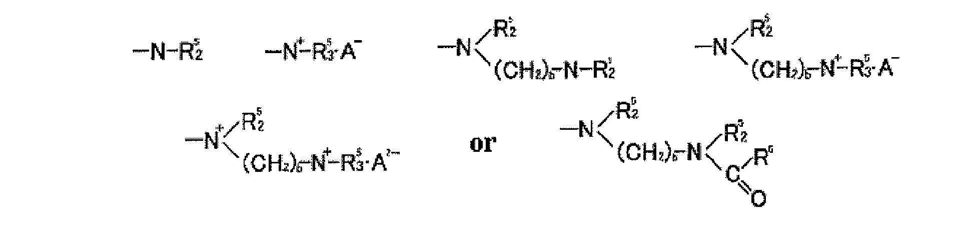 wherein each R⁵ is independently a hydrogen atom or an alkyl group having a carbon number 1 to 30, R⁶ is an alkyl group having a carbon number of 1 to 30, A is a chlorine atom, a bromine atom or an iodine atom, and b is an integer of 2 to 6.

## Description

### RELATED APPLICATIONS

This application claims priority to the Japanese Patent Application 2004-308733 dated on October 22, 2004 and is hereby incorporated with reference for all purposes.

### BACKGROUND OF THE INVENTION

### FIELD OF THE INVENTION

The present invention relates to hair cosmetic composition, and particularly to hair cosmetic composition formulated amino-modified or ammonium-modified silicone.

### PRIOR ART

It is known that hair coloring, bleaching agent, heat with a dryer and excessive brushing may cause hair damages leading to split ends, cut ends and dried hair. Therefore hair cosmetic compositions such as hair shampoo, hair rinse and hair styling agent are formulated silicone compounds for the purpose of protecting and repairing the hair damages mentioned above.

For example, a hair cosmetic composition formulated high-molecular-weight silicone (Japanese published unexamined application No. 63-183,517) and that formulated amino-modified silicone (Japanese published unexamined application No. 4-338,317) are disclosed.

However, the above mentioned hair cosmetic composition formulated high-molecular-weight silicone has not shown sufficient adsorption characteristics to hair, and the durability of the hair protection effect was not always satisfactory. Further, the above mentioned hair cosmetic composition formulated amino-modified silicone has not been able to provide the smooth finish because low-molecular-weight amino-modified silicones (having low degree of polymerization) were used. Furthermore, its stability as a compound was poor and so it had tended to have problems such as smell.

### SUMMARY OF THE INVENTION

With the view to solve those problems of the prior arts mentioned above, the present invention intends to provide a hair cosmetic composition having an excellent hair protection effect and its long durability, and giving a moist feeling, softness and smoothness to the hair.

As the result of taking into consideration these circumstances and doing keen examinations in order to resolve the problems, the present inventors found that it was possible to obtain a hair cosmetic composition having a good absorption characteristic to hair as well as a good hair protection effect, with which the hair has no dryness nor stiff feeling and shows the moist and smooth finish by compounding amino-modified or ammonium-modified silicones having a particular degree of polymerization and a particular ratio of amination, and accomplished the present invention.

Thus, the present invention is a hair cosmetic composition characterized by comprising one or more ammo-modified or ammonium-modified silicones represented by the following formula (I): 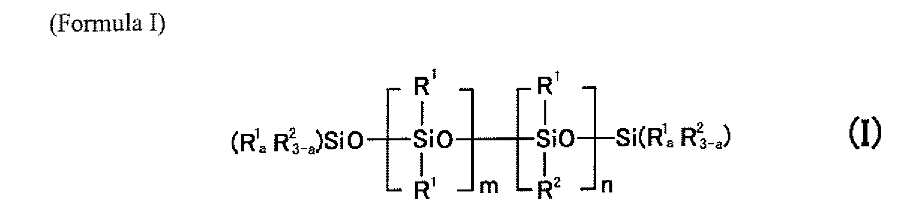 wherein each R¹ is independently a methyl group, a phenyl group or a hydroxyl group, each R² is independently a group shown by a formula R⁴Z, a is an integer of 0 to 3, each of m and n is a positive integer, a mean value of m+n is a range of from 4000 to 6000, and a mean value of n/m is a range of from 0.002 to 0.03, wherein R⁴ is an alkylene group having a carbon number of 3 to 6, and Z is represented by the following formula: 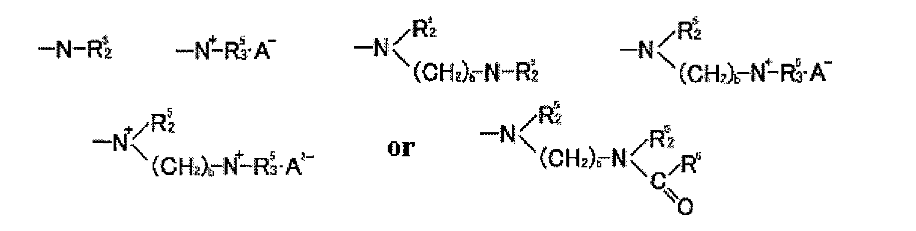 wherein each R⁵ is independently a hydrogen atom or an alkyl group having a carbon number 1 to 30, R⁶ is an alkyl group having a carbon number of 1 to 30, A is a chlorine atom, a bromine atom or an iodine atom, and b is an integer of 2 to 6.

It is preferable that the compounding amount of the amino-modified or ammonium-modified silicone represented by the general formula (I) is from 0.01 to 10 % by mass.

In the hair cosmetic composition described above, it is preferable to further comprise 0.1 to 5.0 % by mass of one or more quaternary ammonium salts represented by the following formula (II): (Formula II)

(NR⁷R⁸₃)⁺X⁻ (II)

wherein R⁷ is an alkyl group or a hydroxyalkyl group having a carbon number of 14 to 22, each R⁸ is independently an alkyl group or a hydroxyalkyl group having a carbon number of 1 to 3 or a benzyl group, and X is a halogen atom or an alkyl sulfate group having a carbon number of 1 to 2.

In the hair cosmetic composition described above, it is preferable to comprise low viscosity liquid oils selected from a group consisting of cyclic silicones, linear silicones and isoparaffinic hydrocarbons.

In the hair cosmetic composition of the present invention, the absorption characteristics to hair and the uniform finishing can be further improved by regulating the polymerization degree and the amination ratio of the compounded amino-modified or ammonium-modified silicone. This gives high hair protection effect and heir conditioning effect (moist feeling, silky, soft and smooth feeling). The durability of these effects can be also obtained.

### DESCRIPTION OF THE PREFERRED EMBODIMENT

### < Amino-modified or ammonium-modified silicone>

Amino-modified or ammonium-modified silicone used for the hair cosmetic composition of the present invention is shown by the following general formula (I): 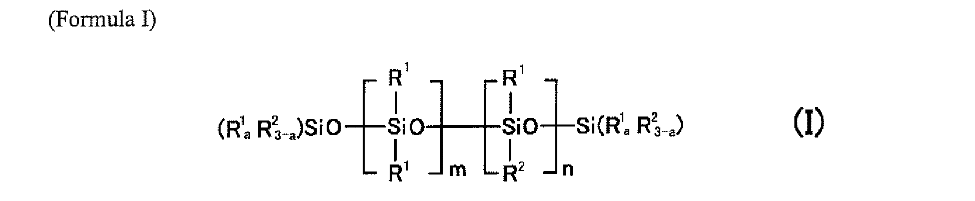 wherein each R¹ is independently a methyl group, a phenyl group or a hydroxyl group, each R² is independently a group shown by a formula R⁴Z, a is an integer of 0 to 3, each of m and n is a positive integer, a mean value of m+n is a range of from 4000 to 6000, and a mean value of n/m is a range of from 0.002 to 0.03, wherein R⁴ is an alkylene group having a carbon number of 3 to 6, and Z is represented by the following formula: 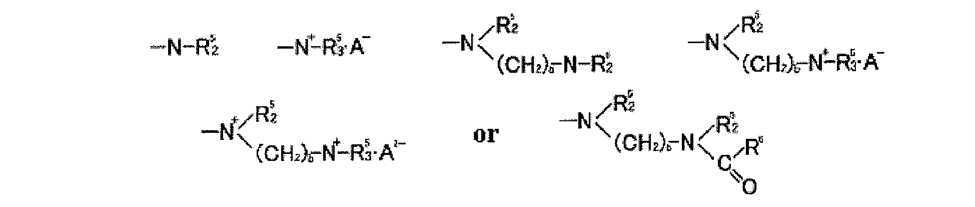 wherein each R⁵ is independently a hydrogen atom or an alkyl group having a carbon number 1 to 30, R⁶ is an alkyl group having a carbon number of 1 to 30, A is a chlorine atom, a bromine atom or an iodine atom, and b is an integer of 2 to 6.

It is preferable that the mean value of m+n is within a range of from 4000 to 6000. If it is less than 4000 (the polymerization degree is low), the hair protection effect is not sufficient and hair becomes sticky. In addition, the stability of the compound itself lowers. If the mean value is more than 6000 (the polymerization degree is high), the finish becomes ununiformly and it gives a stiff feeling to the hair. Further, it may be difficult to compound to cosmetic compositions.

It is preferable that the mean value of n/m is within a range of from 0.002 to 0.03, and more preferable range is 0.005 to 0.01. If it is below 0.002 (the amination ratio is low), the cosmetic composition has a tendency toward showing a bad absorption characteristics to hair and the lowered durability of the hair protection effect. On the other hand, if it is more than 0.03 (the amination ratio is too high), it gives a sticky feeling to the hair.

The above mentioned amino-modified or ammonium-modified silicone can be used by one kind or in combination of more than two kinds. In case that more than two kinds of them are used in combination, it is also necessary that the mean values of m+n and n/m are in the above mentioned range.

Amino-modified or ammonium-modified polymeric silicones can be produced by the same production method as that of general amino-modified or ammonium-modified silicones. For example, it can be produced by performing the polycondensation reaction of γ-aminopropylmethyldiethoxysilane, cyclic dimethylpolysiloxane and hexametyldisiloxane in the presence of an alkali catalyst.

In the present invention, the compounding amount of amino-modified or ammonium-modified silicone is from 0.01 to 10 by mass with respect to the total amount of the cosmetic composition, and preferable range is from 0.1 to 5 % by mass. If it is less than 0.01 % by mass, sufficient hair protection effect and conditioning effect can not be obtained. On the other hand, if it is more than 10 % by mass, the formulation stability deteriorates and the feeling is not good.

### <Low viscosity liquid oil>

Since amino-modified or ammonium-modified silicone usable in the present invention has a high viscosity, it is preferable to dissolve it in low viscosity liquids oil so as to compound it in a hair cosmetic composition. Low viscosity liquid oils include chain silicones, cyclic silicones or isoparaffinic hydrocarbons and the like.

Chain silicone oils include, for example, dimethylpolysiloxane (viscosity 0.65 to 200 cSt/25°C) and the like.

Cyclic silicone oils include octamethylcyclotetrasiloxane, decamethylcyclopentasiloxane, tetradecamethylcyclohexasiloxan and so on.

Further, isoparaffinic hydrocarbons may include those having a boiling point of from 60 to 260 degrees C at normal pressures. They may include, for example, Aisoper A, C, D, E, G, H, K, L and M (trademark) made by Exxon Mobil Corporation, Shellzole 71 (trademark) made by Shell Corporation, and Soltol 100, 130 and 220 (trademark) made by Phillips Corporation.

It is possible to use any one or more low viscosity liquid oils mentioned above. It is preferable that the total compound amount of the low viscosity liquid oils is a range of from 1 to 50 times (by mass) with respect to that of amino-modified or ammonium-modified silicones. It is also preferable that it is a range of from 1 to 90 % by mass with respect to the total amount of the cosmetic composition.

In addition, amino-modified or ammonium-modified silicones and low viscosity liquid oils may be blended separately to a hair cosmetic composition and dissolved in its system.

### < Quaternary ammonium salt >

The hair cosmetic composition of the present invention can show improved absorption characteristics to hair and can give smooth feeling, luster and easiness-to-comb for hair, by blending one or more quaternary ammonium salts represented by the following formula (II): (Formula II)

(NR⁷R⁸₃)⁺X⁻ (II)

wherein R⁷ is an alkyl group or a hydroxyalkyl group having a carbon number of 14 to 22, each R⁸ is independently an alkyl group or a hydroxyalkyl group having a carbon number of 1 to 3 or a benzyl group, and X is a halogen atom or an alkyl sulfate group having a carbon number of 1 to 2.

Alkyl groups having a carbon number of 14 to 22 include, for example, a cetyl group, a stearyl group, a behenyl group and the like. Hydroxyalkyl groups having a carbon number of 14 to 22 include, for example, a 12-hydroxystearyl group and the like. As R⁷, a stearyl group and a behenyl group are particularly preferable. The preferable groups as R⁸ include a methyl group, an ethyl group, a propyl group, a hydroxymethyl group, a hydroxyethyl group. Preferably, X represents a chlorine atom or a bromine atom.

Quaternary ammonium salts represented by the formula (II) include, for example, cetyltrimethylammonium chloride, stearyltrimethylammonium chloride, behenyltrimethylammonium chloride, behenyldimethylhydroxyethylammonium chloride, stearyldimethylbenzyl ammonium chloride, cetyltriethylammonium methylsulfate and the like. Among them, stearyltrimethylammonium chloride, behenyltrimethylammonium chloride, stearyldimethylbenzylammonium chloride and their mixture are particularly preferable.

The compounding amount of the quaternary ammonium salts is a range of from 0.1 to 5.0 % by mass with respect to the total amount of the hair cosmetic composition, and preferable range is from 0.6 to 3.0 % by mass. If it is less than 0.1 % by mass, the effect of addition of the quaternary ammonium salts cannot be sufficiently obtained. On the other hand, if it is more than 5.0 % by mass, the viscosity of the hair cosmetic composition becomes too high and thus it is not preferable.

In the hair cosmetic composition of the present invention, it is possible to obtain a hair cosmetic composition having a more excellent conditioning effect by additionally blending one or more guanidine derivatives represented by the following formula (III): 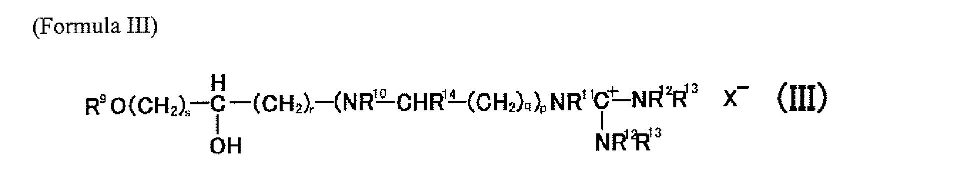 wherein R⁹ represents a linear or branched alkyl group, alkenyl group or alkylether group having a carbon number of 1 to 21, R¹⁰ and R¹¹ represent a hydrogen atom or a linear or branched alkyl group or alkenyl group having a carbon number of 1 to 12 which may be substituted by a hydroxyl group, R¹² and R¹³ represent a hydrogen atom or a linear or branched alkyl group or alkenyl group having a carbon number of 1 to 6 which may be substituted by a hydroxyl group, R¹⁴ represents a hydrogen atom or COOR¹⁵, R¹⁵ represents a hydrogen atom or a linear or branched alkyl group or alkenyl group having a carbon number of 1 to 6 which may be substituted by a hydroxyl group, p represents 0 or 1, and q, r, s represent an integral number of 0 to 9 and X represents an inorganic acid salt such as hydrochloride, bromate, sulfate and phosphate or an organic acid salt such as acetate, tartrate, citrate, p-toluene sulfate, higher fatty acid salt, glycolate, L or DL-pyrrolidone carboxylate, acidic amino acid salt and pyroglutamate.

As a commercial product, the guanidine derivatives mentioned above may include, for example, Amisafe LM A-60 (trademark) made by Ajinomoto Co. Inc. represented by the following formula (IV): 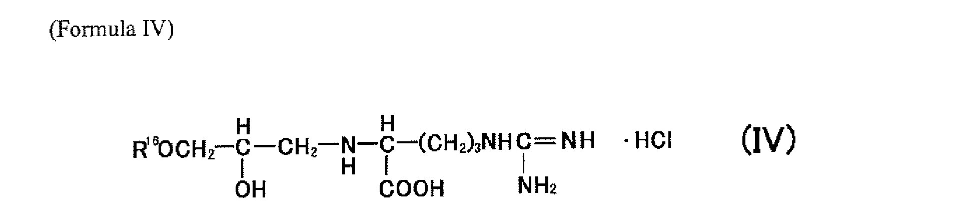 wherein R¹⁶ is a linear or branched alkyl group having a carbon number of 10 to 22.

It is preferable that the compounding amount of the guanidine derivatives mentioned above is a range of from 0.01 to 3.5 % by mass with respect to the total amount of the hair cosmetic composition, and more preferable range is 0.1 to 2.0 % by mass. If it is less than 0.01% by mass, the aimed effects may not be obtained. If it is more than 3.5 % by mass, it may give a heavy feeling to hair.

In the hair cosmetic composition of the present invention, it is preferable to further blend a moisturizing agent.

Mmoisturizing agents include, for example, monosaccharide, disaccharide or oligosaccharide of ethylene glycol, polyethylene glycol, propylene glycol, dipropylene glycol, glycerin, diglycerin, triglycerin, tetraglycerin, 1,3-butylene glycol, 1,4-butylene glycol, xylitol, sorbitol, maltitol, glucose, maltose, sucrose, fructose, maltotriose, threitol, erythritol, starch or decomposed sugar reducing alcohol and their derivatives, chondroitin sulfate, hyaluronic acid, mucoitinsulfuric acid, carronic acid, athero-collagen, cholesteryl -12- hydroxystearate, sodium lactate, dl- pyrrolidone carboxylate, soluble collagen, diglycerin (EO) PO adduct, Chesnut rose extract, Yarrow extract, Yellow sweet clover extract, isoprene glycol, sodium polyglutamate, alkylene oxide derivatives {for, example, POE (14) POP (7) dimethyl ether, POE (10) POP (10) dimethyl ether, POE (6) POP (14) dimethyl ether, POE (25) POP (40) dimethyl ether, POE (25) POP (25) dimethyl ether, POE (7) POP (12) dimethyl ether, POE (22) POP (40) dimethyl ether, POE (35) POP (40) dimethyl ether, POE (45) POP (34) dimethyl ether, POE (50) POP (40) dimethyl ether, POE (55) POP (30) dimethyl ether, POE (30) POP (34) dimethyl ether, POE (25) POP (30) dimethyl ether, POE (27) POP (14) dimethyl ether, POE (55) POP (28) dimethyl ether, POE (36) POP (41) dimethyl ether, POE (7) POP (12) dimethyl ether, POE (17) POP (4) dimethyl ether, POE (14) POB (7) dimethyl ether, POE (10) POP (10) diethyl ether, POE (10) POP (10) dipropyl ether, POE (10) POP (10) dibutyl ether} etc. It is also possible to use one or more of them.

In the above description, POE, POP and POB are abbreviated expressions of polyoxyetylene, polyoxypropylene and polyoxybutyrene, respectively.

Furthermore, as the hair cosmetic composition of the present invention, it is possible to obtain a hair cosmetic composition having more improved hair maintaining-and-repairing-effects, which are kept for a long time, by blending components for hair maintenance and repair such as lecithin; hydrolyzed protein and its derivatives; amino acids such as glutamic acid, arginine, glycine, alanine, serine, proline, leucine and isoleucine; vitamins; ceresin and the like.

In addition to the essential components mentioned above, in the hair cosmetic composition of the present invention, oil contents such as liquid paraffin, squalane, lanoline derivatives, higher alcohol, various ester oils, avocado oil, palm oil, beef tallow, jojoba oil, polyalkylene glycol polyeter and its carboxylic acid oligoester and terpene hydrocarbon oil; ultraviolet absorbers; ultraviolet scattering agents; resins such as acrylic resin, silicone resin and polyvinyl pyrrolidone; proteins and decomposed proteins such as soybean protein, gelatine, collagen, silk fibroin and elastin; preservatives such as ethylpareben and butylparaben; activators such as biotin and pantothenic acid derivatives; blood circulation stimulant such as γ-oryzanol, dextrin sodium sulfate, vitamin E derivatives and nicotinic acid derivatives; antiseborrheic agents such as sulfur and thianthol; diluents such as ethanol, isopropanol, tetrachlorodifluoroethane; thickeners such as carboxyvinyl polymer; chemicals; fragrances and pigments may be blended in accordance with any purpose so far as the quantitative and qualitative range that does not undermine the effect of the present invention.

The hair cosmetic composition of the present invention may take any optional form, such as soluble type, emulsion type, powder dispersion type, double phase type of oil-water or triple phase type of oil-water-powder. In the case of the emulsion type, an oil phase containing amino-modified or ammonium-modified silicones is used by being emulsified with a surfactant. Or, it is possible to produce an emulsion by dissolving a surfactant into water-soluble polyalcohol, adding oil component containing amino-modified or ammonium-modified silicones to prepare an emulsion composition and diluting the composition with water.

As the surfactants, while the quaternary ammonium salt represented by the formula (II) mentioned above may be used, other surfactants may be also used.

Anionic surfactants include, for example, fatty acid soap (for example, sodium laurate, sodium palmitate); higher alkyl sulfate ester salt (for example, sodium lauryl sulfate, potassium lauryl sulfate); alkyl ether sulfate ester salt (for example, POE lauryl sulfate triethanolamine, sodium POE lauryl sulfate); N-acyl sarcosinic acid (for example, sodium lauroyl sarcocinate); higher fatty acid amide sulfonate (for example, sodium N-myristoyl-N-znethyl taurine, sodium coconut oil fatty acid methyl tauride, sodium laurylmethyl tauride); phosphate ester salt (sodium POE oleylether phosphate, POE stearylether phosphate); sulfosuccinate (for example, sodium di-2-ethylhexyl sulfosuccinate, sodium monolauroyl monoethanolamide polyethylene sulfosuccinate, sodium lauryl polypropylene glycol sulfosuccinate); alkylbenzene sulfonate (for example, sodium linear dodecylbenzene sulfonate, triethanolamine linear dodeylbenzene sulfonate, linear dodecylbenzene sulfonate); higher fatty acid ester sulfate ester salt (for example, sodium hydrogenated gryceryl cocoate sulfate), N- acyl glutamate (for example, monosodium N-lauroyl glutamate, disodium N-stearoyl glutamate, monosodium N-myristoyl-L-glutamate); sulfonated oil (for example, Turkey red oil); POE alkyl ether carboxylic acid; POE alkyl aryl ether carboxylate; α-olefine sulfonate; higher fatty acid ester sulfonate, secondary alcohol sulfate ester salt, higher fatty acid alkylolamide sulfate ester salt, sodium lauroyl monoethanolamide succinate; N-palmitoyl asparaginate ditriethanolamine; sodium casein and the like.

Cationic surfactants include, for example, alkyltrimethyl ammonium salt (for example, stearyltrimethyl ammonium chloride, lauryltrimethyl ammonium chloride); alkylpyridinium salt (for example, cetylpyridinium chloride); distearyldimethyl ammonium chloride; dialkyldimethyl ammonium salt; poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride and the like.

Ampholytic surfactants include, for example, imidazoline base ampholytic surfactants (for example, sodium 2-undecyl-N,N,N-(hydroxyethylcarboxymethyl)-2-imidazoline, 2-cocoyl-2-imidazolinium hydroxide-1-carboxyethyloxy)-2-sodium salt; betaine base surfactants (for example, 2-heptadecyl-N-carboxymethyl-N-hydroxyethylimidazolinium betaine, lauryldimethyl aminoacetate betaine, alkyl betaine, amidobetaine, sulfobetaine) and the like.

Lipophilic nonionic surfactants include for example, sorbitan fatty acid esters (for example, sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate); glyceryl polyglyceryl fatty acids (for example, glyceryl monocotton oil fatty acid, glyceryl monoerucate, glyceryl sesquioleate, glyceryl monostearate, glyceryl α, α'-oleate pyroglutamate, glyceryl monostearate malate); propylene glycol fatty acid esters (for example, propylene glycol monostearate); hydrogenated caster oil derivative; glyceryl alkyl ether and the like.

Hydrophilic nonionic surfactants include, for example, POE sorbitan fatty acid esters (for example, POE sorbitan monooleate, POE sorbitan monostearate, POE sorbitan monooleate, POE sorbitan tetraoleate); POE sorbit fatty acid esters (for example, POE sorbit monolaurate, POE sorbit monooleate, POE sorbit pentaoleate, POE sorbit monostearate), POE glyceryl fatty acid esters (for example, POE monooleate such as POE glyceryl monostearate, POE glyceryl monoisostearate, POE glyceryl triisostearate); POE fatty acid esters (for example, POE distearate, POE monodioleate, ethyleneglycol distearate); POE alkyl ethers (for example, POE lauryl ether, POE oleyl ether, POE stearyl ether, POE behenyl ether, POE-2-octyldodecyl ether, POE cholestanol ether); puluronic types (for example, Puluronic), POE/POP alkyl ethers (for example, POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (for example, Tetronic); POE castor oil hydrogenated castor oil derivatives (for example, POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivatives (for example, POE sorbitol beeswax); alkanolamides (for example, coconut oil fatty acid diethanolamide, lauric acid monoethanolamide, fatty acid isopropanolamide); POE propyleneglycol fatty acid esters; POE alkyl amines; POE fatty acid amides; sucrose fatty acid esters; alkylethoxydimethylamine oxide; trioleyl phosphoric acid and the like.

The hair cosmetic composition of the present invention means any type of cosmetics used on hair, and they widely include so-called hair cleansing agents such as shampoo, and so-called hair treating agents such as hair rinse, hair conditioner, hair treatment, hair pack, hair spray and hair styling agent. In addition, the hair cosmetic composition may take any type of usage including that of washing (rinsing) or not washing away after applying and impregnating it to the whole hair.

The present invention will be described in more detail in the following examples. However, these examples will not limit the scope of the present invention. The compounding amount is represented in the percentage by mass if not otherwise specified.

### EXAMPLE 1

Hair cosmetic compositions (hair oils) shown in Table 1 were evaluated according to the following evaluation criteria.

### Evaluation 1: Absorption characteristics

Hair samples (N=10) to which each test portion had been applied were observed by the EDX (about X1000). The mass concentration (%) ratio of Si atoms to S atoms found largely in hair (X=Si/S) was calculated to evaluate the absorption characteristics according to the following criterion.
A: X ≥ 0.25
B: 0.2 ≤ X < 0.25
C: 0.15 ≤ X < 0.2
D: 0.05 ≤ X < 0.15
E: X < 0.05

### Evaluation 2: Moist feeling (no dry feeling)

About the moist feeling (no dry feeling) of the hair after application of each test portion, the sensory evaluation was conducted by 20 specialized panelists. The criterion is shown as follows;
A: More than 16 of 20 panelists answered that it had the moist feeling.
B: 13 to 16 of 20 panelists answered that it had the moist feeling.
C: 8 to 12 of 20 panelists answered that it had the moist feeling.
D: Less than 8 of 20 panelists answered that it had the moist feeling.

### Evaluation 3: Softness (no stiffness)

About the softness (no stiffness) of the hair after application of each test portion, the sensory evaluation was conducted by 20 specialized panelists. The evaluation criterion is shown as follows;
A: More than 16 of 20 panelists answered that it was soft.
B: 13 to 16 of 20 panelists answered that it was soft.
C: 8 to 12 of 20 panelists answered that it was soft.
D: Less than 8 of 20 panelists answered that it was soft.

### Evaluation 4: Smoothness (no sticky feeling)

About the smoothness (no sticky feeling) of the hair after application of each test portion, the sensory evaluation was conducted by 20 specialized panelists. The criterion is shown as follows;
A: More than16 of 20 panelists answered that it was smooth.
B: 13 to 16 of 20 panelists answered that it was smooth.
C: 8 to 12 of 20 panelists answered that it was smooth.
D: Less than 8 of 20 panelists answered that it was smooth.

**(Table 1)**

| FORMULATION | | EXAMPLE | | | | | | |
|---|---|---|---|---|---|---|---|---|
| | | 1 | 2 | 3 | 4 | 5 | 6 | 7 |
| | Amino-modified silicone 1 R¹=CH₃, R²=(CH₂)₃N(CH₃)(CH₂)₂N(CH₃)₂ m=5000, n=25, m+n=5025, n/m=0.005 | 0.1 | 1 | - | - | 0.01 | - | - |
| | Amino-modified silicone 2 R¹=CH₃ R²=(CH₂)₃N(CH₃)(CH₃)₂N(CH₃)₂ m=5000, n=150, m+n=5150, n/m=0.03 | - | - | 1 | - | - | - | - |
| | Amino-modified silicone 3 R¹=CH₃ R²=(CH₂)₃N(CH₃)(CH₂)₂N(CH₃)₂ m=10000, n=15, m+n=10015, n/m=0.0015 | - | - | - | 1 | - | - | - |
| (1) | Amino-modified silicone 4 R¹=CH₃, R²(CH₂)₃N(CH₃)(CH₃)₂N(CH₃)₂ m=10000, n=75, m+n=10075. n/m=0.0075 | - | - | - | - | 1 | - | - |
| | Amino-modified silicone 5 R¹=CH₃, R²=(CH₂)₃N(CH₃)₂N(CH3)₂ m=5000, n=6, m+n=5005, n/m=0.001 | - | - | - | - | - | 1 | - |
| | Amino-modified silicone 6 R¹=CH₂, R²=(CH₂)₃N(CH₃)(CH₂)₂N(CH₃)₂ m=2OO. n=10. m+n=210, n/m=0.05 | - | - | - | - | - | - | 1 |
| (2) | Light liquid Isoparaffin Dimethylsilicone (5cs) | Balance | Balance | Balance | Balance | Balance | Balance | Balance |
| (3) | | 5 | 5 | - | 5 | 10 | 5 | 5 |
| (4) | Fragrance | Appropriate amount | Appropriate amount | Appropriate amount | Appropriate amount | Appropriate amount | Appropriate amount | Appropriate amount |
| (5) | Pigments | Appropriate amount | Appropriate amount | Appropriate amount | Appropriate amount | Appropriate amount | Appropriate amount | Appropriate amount |
| Absorption characteristics | | B | A | A | C | D | D | B |
| Moist feeling (no dry feeling) | | A | A | A | B | C | B | B |
| Softness (no stiffness) | | A | A | A | B | C | B | A |
| Smoothness (no sticky feeling) | | A | A | A | B | B | B | C |

### (Manufacturing process)

Amino-modified silicones (1) are added to a mixture of light liquid isoparaffin (2) and dimethylsilicone (3) and dissolved, and then fragrance (4) and pigments (5) are added.

As shown in Table 1, test-examples 1 to 3 formulated amino-modified silicones, which have m+n range within from 4000 to 6000 and the n/m range within from 0.002 to 0.03, showed the good absorption characteristics to hair and the excellent conditioning effects. On the other hand, in test example 7 which used amino-modified silicone having the m+n range less than 4000, the smooth finish was not obtained.

Further, in test examples 4 to 6 which used amino-modified silicones having the n/m range less than 0.002, the absorption characteristics to hair were not good and the continuousness of the effects was not obtained. In addition, in test example 5, although ammo-modified silicone 1 which independently has the values of m+n and n/m within the range described above was used, the effects were not obtained since the overall mean value was out of the range.

### EXAMPLE 2

Hair cosmetic compositions (hair rinses) shown in Table 2 were evaluated according to the following evaluation criteria.

### Evaluation 1: Uniform finishing

Hair samples (N=10) to which each test portion had been applied, washed away and then dried were observed by a frictional force microscope. 20 specialized panelists visually judged the uniformity of the distribution of low friction area on the obtained images in comparison with the healthy hair, and evaluated the uniform finishing. In addition, the virgin hair was used as healthy hair, that had the uniformly low friction and showed almost no distribution. The criterion is shown as follows;
A: More than 16 of 20 panelists answered that it was at the same level.
B: 13 to 16 of 20 panelists answered that it was at the same level.
C: 8 to 12 of 20 panelists answered that it was at the same level.
D: Less than 8 of 20 panelists answered that it was at the same level.

### Evaluation 2: Moist feeling (no dry feeling)

About the moist feeling (no dry feeling) of the hair after application of each test portion, the sensory evaluation was conducted by 20 specialized panelists. The criterion is shown as follows;
A: More than 16 of 20 panelists answered that it had the moist feeling.
B: 13 to 16 of 20 panelists answered that it had the moist feeling.
C: 8 to 12 of 20 panelists answered that it had the moist feeling.
D: Less than 8 of 20 panelists answered that it had the moist feeling.

### Evaluation 3: Softness (no stiffness)

About the softness (no stiffness) of the hair after application of each test portion, the sensory evaluation was conducted by 20 specialized panelists. The criterion is shown as follows;
A: More than 16 of 20 panelists answered that it was soft.
B: 13 to 16 of 20 panelists answered that it was soft.
C: 8 to 12 of 20 panelists answered that it was soft.
D: Less than 8 of 20 panelists answered that it was soft.

### Evaluation 4: Smoothness (no sticky feeling)

About the smoothness (no sticky feeling) of the hair after application of each test portion, the sensory evaluation was conducted by 20 specialized panelists. The criterion is shown as follows;
A: More than 16 of 20 panelists answered that it was smooth.
B: 13 to 16 of 20 panelists answered that it was smooth.
C: 8 to 12 of 20 panelists answered that it was smooth.
D: Less than 8 of 20 panelists answered that it was smooth.

### Evaluation 5: (Easiness for passage of fingers on application)

Each test portion was applied to the hair and the sensory evaluation about the easiness for passage of fingers at the time of application was conducted by 20 specialized panelists. The criterion is shown as follows;
A: More than 16 of 20 panelists answered that it was easy to pass fingers.
B: 13 to 16 of 20 panelists answered that it was easy to pass fingers.
C: 8 to 12 of 20 panelists answered that it was easy to pass fingers.
D: Less than 8 of 20 panelists answered that it was easy to pass fingers.

### Evaluation 6: (Smoothness at the time of rinsing)

Each test portion was applied to the hair and the sensory evaluation about the smoothness at the time of rinsing was conducted by 20 specialized panelists. The criterion is shown as follows;
A: More than 16 of 20 panelists answered that it was smooth.
B: 13 to 16 of 20 panelists answered that it was smooth.
C: 8 to 12 of 20 panelists answered that it was smooth.
D: Less than 8 of 20 panelists answered that it was smooth.

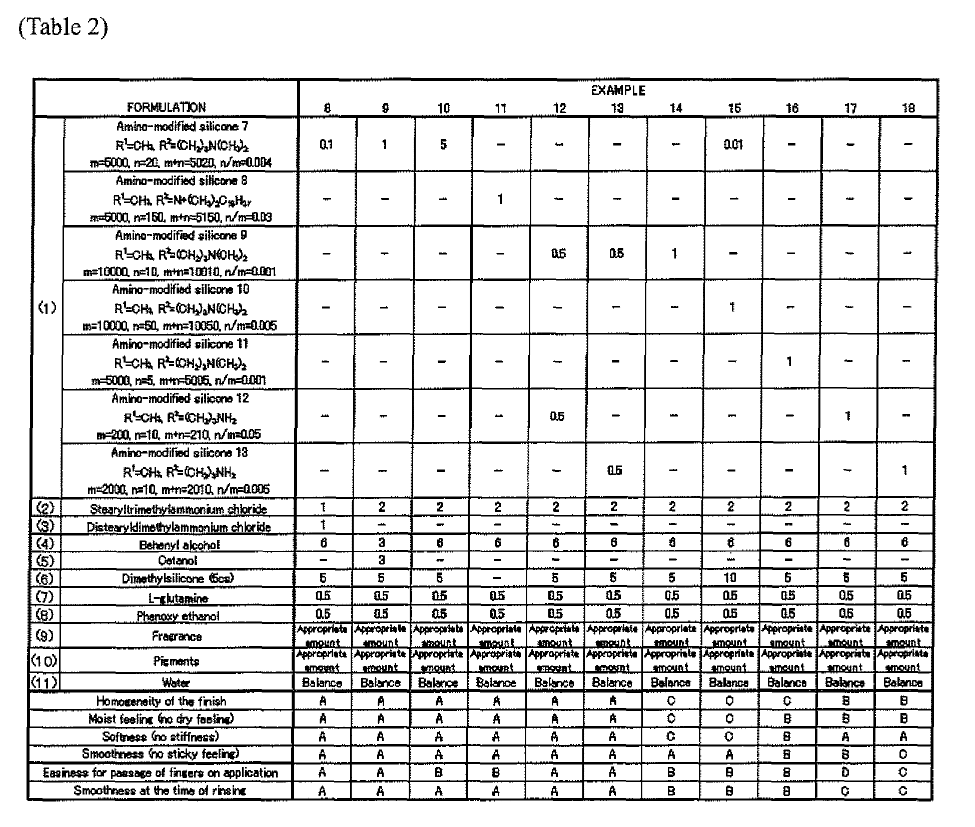

### (Manufacturing process)

1. Stearyltrimethylammonium chloride (2) and distearyldimethylammonium chloride (3) are dissolved in benyl alcohol (4) and cetanol (5).
2. Amino-modifeid silicones (1) are dissolved in dimethyl silicone (6).
3. The mixture of Step 2 is added to that of Step 1 and emulsified. And then, materials (7) to (11) are added to said emulsion and mixed.

As shown in Table 2, test examples 8-13 which are hair cosmetic compositions of the present invention containing amino-modified silicones with the m+n range of from 4000 to 6000 and n/m range of from 0.002 to 0.03 showed the good absorption characteristics to hair and the excellent conditioning effects. As shown by test examples 12 and 13, when a number of amino-modified silicones was formulated, even if each of them independently had the values which are out of the range described above, the effects were obtained, as long as the mean values of all silicones were within the above range.

On the other hand, test examples 14-16 using the amino-modified silicone with the value of m+n more than 6000 or the value of n/m less than 0.002 were inferior in the uniform finishing. In addition, although test example 15 contained the amino-modified silicone 7 which independently has the values of m+n and n/m within the above range, the sufficient effects were not obtained since the mean values were out of the range.

Further, test examples 17 and 18 using the amino-modified silicone with the value of m+n less than 4000 were inferior in the sense of use or showed the sticky finish.

Prescription examples will be shown as follows. All of the hair cosmetic compositions described below were excellent in the hair protection effect and its durability. In addition, they gave the moist feeling, the softness and the smoothness to the hair.

### Prescription example 1 (Hair oil)

| (Formulation) | (% by mass) |
|---|---|
| (1) Light liquid isoparaffin | 85 |
| (2) Amino-modified polymeric silicone (represented by the general formula (I) in which; | 2 |
| - R¹ is a methyl group | |
| - R² is -(CH₂)₃N(CH₃)(CH₂)₂N(CH₃)₂, and | |
| - m = 4000 and n=20) | |
| (3) Methylphenylpolysilixane | 5 |
| (4) Fregrance | Appropriate amount |

### (Manufacturing process)

The materials (1) to (3) are blended. After adding fragrance (4), the mixture is stirred to be homogeneous.

### Prescription example 2 (Hair spray)

| (Formulation) | (% by mass) |
|---|---|
| (1) Volatile isoparaffin | 16 |
| (2) Dimethylpolysiloxane | 7 |
| (3) Propylene glycol | 5 |
| (4) Ethanol | Appropriate amount |
| (5) Purified water | 2 |
| (6) Amino-modified polymeric silicone (represented by the general formula (I) in which; | 4 |
| - R¹ is a methyl group | |
| - R² is -(CH₂)₃N(CH₃)₂, and | |
| - m = 4000 and n=20) | |
| (7) Poly (oxyethylene/oxypropylene)/methylpolysiloxane copolymer | 4 |
| (8) Fragrance | Appropriate amount |
| (9) LPG | Balance |

### (Manufacturing process)

A mixture of materials (1) and (2) is added to a mixture of materials (3) to (7) and emulsified. After adding fragrance (8), the mixture is stirred to be homogeneous. Then, both the mixture and LPG (9) fill a container.

### Prescription example 3 (Hair mousse (aerosol))

| (Formulation) | (% by mass) |
|---|---|
| (1) Ethanol | 5 |
| (2) Dimethylpolysiloxane | 5 |
| (3) Amino-modified silicone (represented by the general formula (I) in which; | 5 |
| - R¹ is a methyl group | |
| - R² is -(CH₂)₃NH₂, and | |
| - m = 5000 and n=10) | |
| (4) Volatile isoparaffin | 25 |
| (5) 1,3-butylene glycol | 5 |
| (6) Isostearic acid | 1 |
| (7) Polyoxyethylene hydrogenated caster oil | 1 |
| (8) 2-alkyl-N-carboxymethyl-N-hydroxyethylimidazolinium betaine | 1 |
| (9) Phenoxyethanol | Appropriate amount |
| (10) Purified water | Balance |
| (11) Fragrance | Appropriate amount |

### (Manufacturing process)

A mixture of materials (1) to (4) and (11) is added to a mixture of materials (5) to (10) and emulsified.

### Prescription example 4 (Hair cream)

| (Formulation) | (% by mass) |
|---|---|
| (1) Volatile isoparaffin | 10 |
| (2) Dimethylethylpolysiloxane | 1 |
| (3) Ethanol | 10 |
| (4) 1,3-butylene glycol | 5 |
| (5) POE (14) POP (7) dimethyl ether | 1 |
| (6) Isostearic acid | 0.5 |
| (7) Polyoxyethlene hydrogenated caster oil | 0.1 |
| (8) 2-alkyl-N-carboxymethyl-N-hydroxyethylimidazolinium betaine | 1 |
| (9) Sodium hydroxide | 0.3 |
| (10) Amino-modified silicone (represented by the general formula (I) in which; | 2 |
| - R¹ is a hydroxyl group | |
| - R² is -(CH₂)₃NH₂, and | |
| - m = 5000 and n=10) | |
| (11) Carboxy vinyl polymer | 0.8 |
| (12) Paraoxybenzoic ester | Appropriate amount |
| (13) Edetate trisodium | Appropriate amount |
| (14) Purified water | Balance |
| (15) Fragrance | Appropriate amount |

### (Manufacturing process)

The materials (5) to (12) are blended to prepare a homogenous mixture. And then the materials (1) to (4) and (15) are added to said mixture and emulsified. After adding edentate trisodium (13), the mixture is neutralized with purified water (14) and stirred to produce a homogeneous mixture.

### Prescription example 5 (Hair cream)

| (Formulation) | (% by mass) |
|---|---|
| (1) Liquid paraffin | 5 |
| (2) Vaseline | 2 |
| (3) Dimethylpolysiloxane | 5 |
| (4) Amino-modified silicone (represented by the general formula (I) in which; | 1 |
| - R¹ is a methyl group, | |
| - R² is -(CH₂)₃N⁺(CH)₃C₁₈H₃₇Cl⁻, and | |
| - m = 5000 and n=25) | |
| (5) Cetanol | 4 |
| (6) Stearyl alcohol | 1 |
| (7) 1,3-butylene glycol | 10 |
| (8) Polyoxyethylene/polyoxypropylene dimethylether | 2 |
| (9) Polyoxypropylene glyceryl ether | 2 |
| (10) Polyoxyethylene glyceryl isostearate | 2 |
| (11) Lipophilic glyceryl monostearate | 2 |
| (12) Polymer JR-400 (trade mark) (by Toho Chemical Industries) | 0.5 |
| (13) Hydrolyzed wheat protein | 0.1 |
| (14) Paraoxybenzoic ester | Appropriate amount |
| (15) Purified water | Balance |
| (16) Fragrance | Appropriate amount |

### (Manufacturing process)

The oil layer of (1) to (6) and the water layer of (7) to (15) are dissolved by heating and mixed, respectively. Fragrances (16) are added to the oil layer and homogenized. And then, the two layers are mixed and emulsified.

### Prescription example 6 (Hair cream)

| (Formulation) | (% by mass) |
|---|---|
| (1) Dimethyl polysiloxane | 15 |
| (2) SILWET 236-L (trade mark) (by Nippon Unicar) | 0.1 |
| (3) Polyoxethylene/methylpolysiloxane copolymer | 0.2 |
| (4) Ethanol | 10 |
| (5) Propylene glycol | 5 |
| (6) POE (36) POP (41) dimethyl ether | 1 |
| (7) 2-amino-2-methyl-l-propanol | Appropriate amount |
| (8) Trisodium edentate | Appropriate amount |
| (9) Xanthan gum | 0.1 |
| (10) Vinylacetate/Vinylpyrrolidone copolymer | 0.5 |
| (11) Acrylic acid/alkyl methacrylate copolymer | 0.2 |
| (12) Sodium polyacrylate/sodium acryloyldimethyl taurate copolymer | 0.1 |
| (13) Carboxy vinyl polymer | 0.4 |
| (14) Amino-modified silicone (represented by the general formula (I) in which; | 0.5 |
| - R¹ is a methyl group, | |
| - R² is -(CH₂)₃N (CH₃)(CH₂)₂N(CH₃)C=O(C₂H₅), and | |
| - m = 5000 and n=50) | |
| (15) Purified water | Balance |
| (16) Fragrance | Appropriate amount |

### (Manufacturing process)

A mixture of materials (1) to (4) and (16) is added to a homogeneous mixture of materials (5) to (13) and emulsified. After adding the amino-modified silicone (14), the mixture is neutralized with purified water (15) and then stirred to be homogeneous.

### Prescription example 7 (Hair cream)

| (Formulation) | (% by mass) |
|---|---|
| (1) Liquid paraffin | 10 |
| (2) Vaseline | 3 |
| (3) Dimethylpolysiloxane | 2 |
| (4) Amino-modified silicone (represented by the general formula (I) in which; | 1 |
| - R¹ is a methyl group, | |
| - R² is -(CH₂)₃N (CH₂)₂, and | |
| - m = 5000 and n=25) | |
| (5) Propylene glycol | 10 |
| (6) Polyoxypropylene (40) butyl ether | 2 |
| (7) Pentaerythritol tetra-2-ethylhexanoate | 3 |
| (8) Polyoxyethylene hydrogenated caster oil | 2 |
| (9) Potassium hydroxide | 0.1 |
| (10) Carboxy vinyl polymer | 0.3 |
| (11) Hydroxyethyl polyacrylate/sodium acryloyldimethyl taurate copolymer | 0.1 |
| (12) Purified water | Balance |
| (13) Fragrance | Appropriate amount |

### (Manufacturing process)

A mixture of materials (1) to (6) and (13) is added to a mixture of materials (7) to (10) and emulsified. After adding the copolymer (11) and homogenizing, the mixture is neutralized with purified water (12) and then stirred to be homogeneous.

### Prescription example 8 (Hair cream)

| (Formulation) | (% by mass) |
|---|---|
| (1) Methyl polysilixane | 10 |
| (2) Amino-modified silicone (represented by the general formula (I) in which; | 1 |
| - R¹ is a methyl group, | |
| - R² is -(CH₂)₃NH₂, and | |
| - m = 4000 and n=20) | |
| (3) Polyoxethylene/methylpolysiloxane copolymer | 0.1 |
| (4) Poly (oxyethylene/oxypropylene)/methylpolysiloxane copolymer | 1 |
| (5) Volatile isoparaffin | 2 |
| (6) Cross-linked polyether-modified silicone | 2.5 |
| (7) Dextrin ester | 1 |
| (8) Polyaspartic acid | 0.1 |
| (9) Glycine | 0.1 |
| (10) Ethanol | 5 |
| (11) Paraben | 0.25 |
| (12) Fragrance | Appropriate amount |
| (13) Pigment | Appropriate amount |
| (14) Purified water | Balance |

### (Manufacturing process)

A mixture of materials (8) to (11) and (14) is added to a mixture of materials (1) to (7), (12) and (13) and emulsified.

### Prescription example 9 (Hair lotion)

| (Formulation) | (% by mass) |
|---|---|
| (1) Light isoparaffin | 15 |
| (2) Amino-modified silicone (represented by the general formula (I) in which; | 3 |
| - R¹ is a methyl group, | |
| - R² is -(CH₂)₃N(CH₃)C₂H₅, and | |
| - m = 5000 and n=50) | |
| (3) Dimethyl silicone | 6 |
| (4) 1,3-butylene glycol | 2 |
| (5) Polyoxyethylene (60) hydrogenated caster oil ester | 6 |
| (6) Titanium oxide sol | 10 |
| (7) Ethanol | 15 |
| (8) Purified water | Balance |
| (9) Fragrance | Appropriate amount |

### (Manufacturing process)

A mixture of materials (1) to (3), (8) and (9) is added to a mixture of materials (4) to (7) and emulsified.

### Prescription example 10 (Hair mist)

| (Formulation) | (% by mass) |
|---|---|
| (1) Amino-modified silicone (represented by the general formula (I) in which; | 0.2 |
| - R¹ is a hydroxyl group | |
| - R² is -(CH₂)₃NH₂ and | |
| - m = 4000 and n=120) | |
| (2) Dimethylpolysiloxane | 1 |
| (3) Ethanol | 5 |
| (4) Stearyl alcohol | 0.1 |
| (5) Behenyl alcohol | 0.2 |
| (6) Glycerin | 2 |
| (7) Dipropylene glycol | 1 |
| (8) 1,3-butylene glycol | 1 |
| (9) Alkyltrimethylammonium chloride (77%) | 0.5 |
| (10) Vinylpyrrolidone/N,N-dimethyl aminoethyl methacrylic acid copolymer | 2.5 |
| (11) Arginine | 0.1 |
| (12) Lecithin | 0.1 |
| (13) Octylmethoxy cinnamate | 0.1 |
| (14) Paraoxybenzoic ester | Appropriate amount |
| (15) Purified water | Balance |
| (16) Fragrance | Appropriate amount |

### (Mamufacturing process)

An oil layer formed by mixing of materials (1) to (4) and a water layer formed by mixing of materials (5) to (15) are respectively dissolved by heating, and then the former is added to the latter and emulsified. Fragrances (16) are added to said emulsion and homogenized.

### Prescription example 11 (Hair rinse)

| (Formulation) | (% by mass) |
|---|---|
| (1) Dimethylpolysilixane | 10 |
| (2) Amino-modified silicone (represented by the general formula (I) in which; | 1 |
| - R¹ is a methyl group | |
| - R² is -(CH₂)₃N(CH₃)₂ and | |
| - m = 5000 and n=20) | |
| (3) Hydrogenated rapeseed alcohol | 3 |
| (4) Cetanol | 1.5 |
| (5) Glycerin | 10 |
| (6) Cetyl 2-ethylhexanoate | 2 |
| (7) Alkyltrimethylammonium chloride | 1.5 |
| (8) Distearyldimethylammonium chloride | 0.3 |
| (9) Sorbitol | 2 |
| (10) Glutamic acid | 0.5 |
| (11) Arginine | 0.1 |
| (12) Glycin | 0.1 |
| (13) Citric acid | 0.01 |
| (14) Tocopheryl acetate | 0.05 |
| (15) Paraoxybenzoic ester | Appropriate amount |
| (16) Phenoxy ethanol | Appropriate amount |
| (17) Hydroxyethyl cellulose | 0.05 |
| (18) Purified water | Balance |
| (19) Fragrance | Appropriate amount |

### (Manufacturing process)

Water-soluble components are blended, dissolved by heating and homogenized. To this mixture, a mixture of oil-soluble components dissolved by heating is added and emulsified. After adding fragrance (19) and homogenizing, the emulsion is cooled.

### Prescription example 12 (Shampoo)

| (Formulation) | (% by mass) |
|---|---|
| (1) Dimethylpolysiloxane | 1.5 |
| (2) Amino-modified silicone (represented by the general formula (I) in which; | 0.2 |
| - R¹ is a hydroxyl group | |
| - R² is -(CH₂)₃N(CH₃)₂ and | |
| - m = 5000 and n=20) | |
| (3) Dipropylene glycol | 3 |
| (4) Ethylene glycol distearate | 2 |
| (5) Coconut oil fatty acid monoethanolamide | 2 |
| (6) Sodium lauroyl methyltaurate | 0.1 |
| (7) Sodium polyoxyethylene lauryl ether sulfate | 7.5 |
| (8) Triethanolamine polyoxyethylene lauryl ether sulfate | 3.5 |
| (9) Coconut oil fatty acid amidopropyl betaine | 3.5 |
| (10) MERQUAT 550 (trade mark) (by Calgon Corporation) | 7.5 |
| (11) Citric acid | 0.01 |
| (12) L-glutamine | 0.2 |
| (13) Sodium chloride | 1 |
| (14) Sodium benzoate | Appropriate amount |
| (15) Disodium edentate | Appropriate amount |
| (16) Sodium hydroxide | 0.01 |
| (17) Purified water | Balance |
| (18) Fragrance | Appropriate amount |

### (Manufacturing process)

MERQUAT 550 (10) is added to purified water (17), dissolved by heating, and homogenized. The materials (1) to (9) are added to the dissolved materials and stirred to be homogeneous. After stopping heating, the materials (11) to (16) and (18) are added to said mixture and the mixture is stirred to be homogeneous.

### Prescription example 13 (Hair conditioner)

| (Formulation) | (% by mass) |
|---|---|
| (1) Isopentyl diol | 5.0 |
| (2) Sorbitol | 5.0 |
| (3) Dimethicone (1,000,000 cSt) | 1.5 |
| (4) Dimethicone (20 cSt) | 2.0 |
| (5) Stearyl alcohol | 3.0 |
| (6) Behenyl alcohol | 2.0 |
| (7) Stearyltrimonium chloride | 1.5 |
| (8) Cetyl isooctate | 1.0 |
| (9) Aminopropyl dimethicone (m=4000, n=120) | 0.2 |
| (10) Arginin | 0.01 |
| (11) PEG-90M | 0.02 |
| (12) Stearyl dihydroxypropyldimonium oligosaccharide | 0.05 |
| (13) Citric acid | 0.07 |
| (14) Phenoxy ethanol | Appropriate amount |
| (15) Mixed fragrance | Appropriate amount |
| (16) Soy lecithin | 0.1 |
| (17) Hydrolyzed wheat protein/hydrolyzed wheat starch | 0.02 |
| (18) Di(phytosteryl/2-octyldodecyl) N-lauroyl glutamate | 0.1 |
| (19) Purified water | Balance |

### (Manufacturing process)

Water-soluble components are dissolved by heating and homogenized. To this mixture, a mixture of oil-soluble components dissolved by heating is added and emulsified. And then, said emulsion is cooled.

### Prescription example 14 (Hair treatment)

| (Formulation) | (% by mass) |
|---|---|
| (1) 1,3-buthylene glycol | 10.0 |
| (2) Dimethicone (1,000,000 cSt) | 3.5 |
| (3) Dimethicone (100 cSt) | 5.0 |
| (4) Sorbitol | 7.0 |
| (5) Hydrogenated rapeseed alcohol | 6.0 |
| (6) Behentrimonium chloride | 3.0 |
| (7) Glyceryl oleate | 1.5 |
| (8) Aminopropyl dimethicone (m=5500, n=150) | 0.5 |
| (9) Glutamic acid | 0.25 |
| (10) Creatine | 0.1 |
| (11) Taurine | 0.1 |
| (12) N-methyl taurate | 0.1 |
| (13) Red algae BG extract | 0.01 |
| (14) PEG-70M | 0.02 |
| (15) Octyl dodecanol | 0.1 |
| (16) Cetanol | 0.1 |
| (15) Ethanol | 0.1 |
| (18) Phenoxyethanol | 1.0 |
| (19) Mixed fragrance | Appropriate amount |
| (20) Honey extract | 0.01 |
| (21) L-menthol | 0.15 |
| (22) Capsicum tincture | 0.1 |
| (23) Soy lecithin | 0.01 |
| (24) Hydrolyzed wheat protein/hydrolyzed wheat starch | 0.02 |
| (25) Di(phytosteryl/2-octyldodecyl) N-lauroyl glutamate | 0.1 |
| (26) Trehalose | 1.0 |
| (27) Alkyl(C12, 14)oxyhydroxy propyl arginine hydrochloride | 0.2 |
| (28) Squalane | 1.0 |
| (29) Sodium pyrrolidone carboxylate | 0.1 1 |
| (30) Pyrrolidone carboxylic acid | 0.2 |
| (31) Royal jelly extract | 0.01 |
| (32) Purified sesame oil | 0.1 |
| (33) Polyquaternium-64 | 0.5 |
| (34) Quaternium-80 | 3.0 |
| (35) Lavender extract | 0.01 |
| (36) Purified water | Balance |

### (Manufacturing process)

Water-soluble components are dissolved by heating and homogenized. To this mixture, a mixture of oil-soluble components dissolved by heating is added and emulsified. And then, said emulsion is cooled.

## Claims

1. A hair cosmetic composition comprising one or more amino-modified or ammonium-modified silicones represented by the following formula (I): 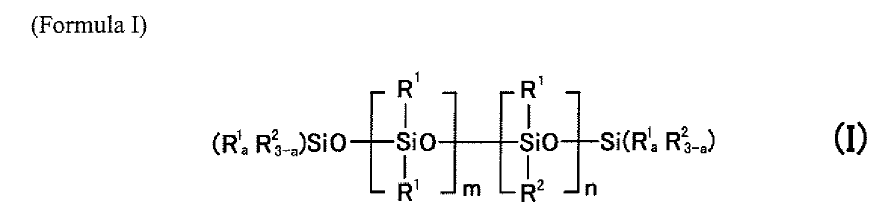 wherein each R¹ is independently a methyl group, a phenyl group or a hydroxyl group, each R² is independently a group shown by a formula R⁴Z, a is an integer of 0 to 3, each of m and n is a positive integer, a mean value of m+n is a range of from 4000 to 6000, and a mean value of n/m is a range of from 0.002 to 0.03,
wherein R⁴ is an alkylene group having a carbon number of 3 to 6, and Z is represented by the following formula: 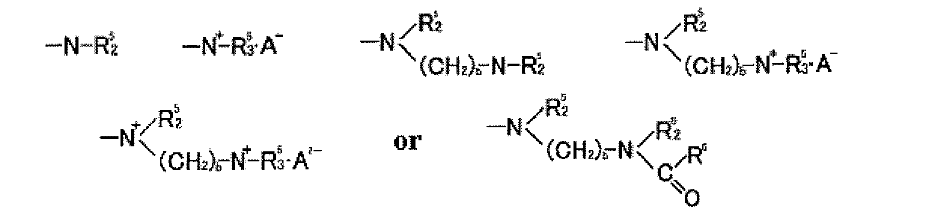 wherein each R⁵ is independently a hydrogen atom or an alkyl group having a carbon number 1 to 30, R⁶ is an alkyl group having a carbon number of 1 to 30, A is a chlorine atom, a bromine atom or an iodine atom, and b is an integer of 2 to 6.

2. The hair cosmetic composition according to claim 1, wherein the compounding amount of the amino-modified or ammonium-modified silicone represented by the general formula (I) is from 0.01 to 10% by mass.

3. The hair cosmetic composition according to claim 1 or 2, further comprising 0.1 to 5.0 % by mass of one or more quaternary ammonium salts represented by the following formula (II): (Formula II)
(NR⁷R⁸₃)⁺X⁻ (II)
wherein R⁷ is an alkyl group or a hydroxyalkyl group having a carbon number of 14 to 22, each R⁸ is independently an alkyl group or a hydroxyalkyl group having a carbon number of 1 to 3 or a benzyl group, and X is a halogen atom or an alkyl sulfate group having a carbon number of 1 to 2.

4. The hair cosmetic composition according to any of claims 1 to 3, wherein comprising low viscosity liquid oils selected from a group consisting of cyclic silicones, linear silicones and isoparaffinic hydrocarbons.
